(19) **Europäisches Patentamt**
**European Patent Office**
**Office européen des brevets**

(11) **EP 3 942 937 A1**

(12) **EUROPEAN PATENT APPLICATION**

(43) Date of publication:
**26.01.2022 Bulletin 2022/04**

(21) Application number: **21183434.6**

(22) Date of filing: **02.07.2021**

(51) International Patent Classification (IPC):
*A23J 3/22* (2006.01)   *A23J 1/00* (2006.01)
*A23L 13/40* (2016.01)   *A23L 31/00* (2016.01)
*C12N 1/14* (2006.01)

(52) Cooperative Patent Classification (CPC):
**A23J 3/227; A23J 1/008; A23L 13/424;**
**A23L 13/46; A23L 31/00; C12N 1/14;**
C12R 2001/845

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **03.07.2020 SE 2050845**
**22.01.2021 SE 2150071**

(71) Applicant: **Mycorena AB**
**415 02 Göteborg (SE)**

(72) Inventors:
• **HÜTTNER, Silvia**
**417 08 Göteborg (SE)**

• **GONÇALVES TEIXEIRA, Paulo**
**414 82 Göteborg (SE)**
• **PERSSON, Frida**
**504 46 Borås (SE)**
• **BALACHANDRAN NAIR, Ramkumar**
**421 41 Västra Frölunda (SE)**
• **JOHANSSON, Anton**
**415 14 Göteborg (SE)**
• **FRÖLING, Ebba**
**412 68 Göteborg (SE)**

(74) Representative: **Valea AB**
**Box 7086**
**103 87 Stockholm (SE)**

(54) **A FOOD PRODUCT COMPRISING A PURE FUNGI BIOMASS**

(57) The present disclosure relates to a food product comprising a pure fungi biomass, the pure fungi biomass comprising fungi of a species belonging to the genus R*hizopus,* wherein the dry weight of the food product comprises within the range of from 10% to 100% of dry weight of the fungi biomass the fungi biomass comprising fungi biomass fibers, wherein 50% or more, such as 70% or more, or 80% or more of the fungi biomass fibers are aligned substantially in planes extending in a first direction. The present disclosure also relates to a method for producing the food product and use of a pure fungi biomass for preparing a food product.

FIG.1

EP 3 942 937 A1

**Description**

TECHNICAL FIELD

The present disclosure pertains to a food product comprising a pure fungi biomass. In particular, this disclosure pertains to a food product comprising a pure fungi biomass comprising fungi of a species belonging to the genus *Rhizopus.* This disclosure also relates to a method for producing the food product and the use of a pure fungi biomass for preparing a food product.

BACKGROUND

Poor nutrition is an issue that exists all over the world, and is manifested not only in increasing levels of starvation and undernourishment, but also in high prevalence of obesity and other forms of malnutrition. These issues are often linked to poverty as well as to deficiencies in both understanding and practices, but also to poor sanitation and regional problems of food security. The latter is also aggravated by soil degradation caused by rampant climate volatility, which severely hampers the ability to grow crops and raise livestock for food production. These issues are particularly pressing in relation to protein, as it is not only the most important macronutrient for health and wellbeing, but also because current methods for production of animal-based protein have been linked to severe negative impact for the climate. To ensure adequate food security without further harming the environment, new solutions for the production of nutritious, abundant, and environmentally friendly protein that is not animal-based will be required.

One suggested solution has been to use mycoprotein, i.e. protein derived from fungi that are produced for the purpose of human consumption. Mycoprotein and its various fermentation-based production methods have a range of advantages and characteristics that make them highly suitable for solving present challenges related to poor nutrition, food security and climate change. Consumption of mycoprotein is associated with a range of benefits to health and wellbeing, attributable to its beneficial nutritional composition.

*Rhizopus* is a genus of fungi that was classified in the 1820s which comprises at least eight separate species. Commercially relevant application areas range from production of industrially relevant enzymes, organic acids and esters with *Rhizopus oryzae* to production of fumaric acid and cortisone using *Rhizopus stolonifer.* Several species have also been used for producing foods, most notably in the production of two types of Javan staple foods - tempeh and black oncom. Tempeh is produced by fermenting whole soybeans to bind them into a cake form. Oncom, by comparison, is made by fermenting various byproducts from other food production processes such as soy pulp, press cakes from peanut or coconut, or cassava tailings.

However, known food products comprising *Rhizopus* consists only to a limited degree of the fungus, with major parts of the weight of the fungi biomass and thus the final products' being made up of the fermentable substrate, i.e. non-pure fungi biomass. One associated issue is thus that more allergens might be present that stem from the other component of the food product, besides mycoprotein, e.g. soy or peanut. Since a major part constitutes of fermentable substrate, the nutritional value of the final food product is dominated by the substrate.

The food production methods with *Rhizopus* use fermentation that takes place in open cultures, where cultivation takes several days to complete. This puts the production at high risk of contamination from other microorganisms that may obstruct the fermentation process or even make the food product unsafe for consumption. Moreover, the long time required for cultivation is ill-adapted for large-scale production, as competitiveness in global food markets require high speed and short downtimes.

Currently, *Rhizopus* -based food products involves so-called solid-state fermentation, signifying that the fungus is directly applied to a solid fermentable substrate. The main drawback of this process is that it is inherently more difficult to scale to industrial levels while maintaining tightly controlled conditions, making it unsuitable for large-scale manufacturing and commercial sales.

There is thus still need for a food product with a non-animal based, high-nutritious and environmentally friendly food product which is suitable for large-scale manufacture and which also has a taste and consistence resembling meat-based food products.

SUMMARY

One or more of the above objects may be achieved with a food product in accordance with claim 1, a method for producing the food product in accordance with claim 10 and/or use of a fungi biomass according to claim 22. Further embodiments are set out in the dependent claims and in the following description.

According to a first aspect, the food product as disclosed herein comprises a pure fungi biomass. The pure fungi biomass comprises fungi of a species belonging to the genus *Rhizopus,* wherein the dry weight of the food product comprises within the range of from 10% to 100% of dry weight of the pure fungi biomass. The fungi biomass comprises fungi biomass fibers and wherein 50% or more, such as 70% or more, or 80% or more of the fungi biomass fibers are aligned in planes extending in a first direction.

The fungi biomass fibers thus substantially extend in the same first direction and in parallel planes.

Consumption of mycoprotein is associated with a range of benefits to health and wellbeing. Fungi biomass comprising fungi of a species belonging to the genus *Rhizopus* has been found particularly advantageous in its content of dietary protein, dietary fiber, essential amino acids, vitamins and minerals and polyunsaturated fatty acids, along with its low levels of sodium, saturated fat and cholesterol. The pure fungi biomass also has a high resemblance with meat-based food products, has a pleasant consistency and taste making it highly suitable to be included in food products. *Rhizopus* also has a high growth rate and can be cultivated in continuous fermentation, meaning that production can be made both highly quick and efficient. The food product according to the present disclosure is furthermore based on pure fungi biomass and thus has a high degree of the fungus Rhizopus which has been seen to provide excellent nutritional value, since the nutritional value of the food product is dominated by the fungus and not the substrate which is advantageous due to excellent nutritional quality and overall suitability for human consumption of mycoprotein and particularly from the fungi of a species belonging to the genus *Rhizopus* .

With "pure fungi biomass" herein is meant that the fungi biomass does not include any residual raw materials/substrates from the cultivation process of the fungi, except for tiny traces such as 1% by weight or less, 0.5% by weight or less, or 0.1% by weight or less, such as 0.05% by weight or less. The food product disclosed herein does therefore not include any residual raw materials/substrates from the cultivation process of the fungi. The fungi cells naturally form a fiber structure, which are here referred to as "fungi biomass fibers".

The food product as disclosed herein may thus be free from fermented ingredients or raw materials.

The fact that the fungi biomass fibers are aligned in planes extending in a first direction, i.e. in parallel planes, provides the fungi biomass with a lamellar-like structure which resembles that of meat.

The dry weight of the food product may comprise within the range of from 25% to 100% of dry weight of the pure fungi biomass.

The dry weight of the food product may comprise within the range of from 50% to 100% of dry weight of the pure fungi biomass, or within the range of from 75% to 95% of weight of the pure fungi biomass.

Since the food product is based on the pure fungi biomass, i.e. without significant parts of the weight of the final products being made up of fermented substrate used, the amount of allergens possibly coming from the substrate are held on a low level and the manufacturing method is made simple since separation of biomass and substrate may be time-consuming and also generative of large amounts of food waste.

The fungi may be of the species *Rhizopus oryzae, Rhizopus microsporus var. oligosporus, Rhizopus arrhizus, Rhizopus delemar, or Rhizopus stolonifer.* These species have been found particularly advantageous in that they have a high growth rate in both liquid and solid state cultivation and a wide substrate flexibility in that they can be grown on many different carbohydrates. Their biomass has a very beneficial composition of macronutrients and a fibrous, meat-like texture. Furthermore, several of these fungal species have been used for centuries in the production of fermented soy and peanut food products South East, and therefore their safety for human consumption is certain.

The amount of protein in the total fungi biomass (dry weight) may be within the range of from 30% to 70%.

The amount of essential amino acids in the fungi biomass (dry weight) may be within the range of from 30% to 70% of the total amino acid content.

The pure fungi biomass as disclosed herein has been found particularly rich in and with an unusually favorable amino acid composition, with a low carbohydrate content and low fat content. The fungi biomass produced may have a minimum protein content of 30%, and a protein content of 30-70% (dry weight) after harvesting, a fiber content of 5-12% (dry weight) after harvesting, a fat content of 1-10% (dry weight) after harvesting and a carbohydrate content of 0.5-5% (dry weight) after harvesting. The content of essential amino acids may be 30-70 g/100 g of the total protein content of the fungi biomass. The values for fatty acids in dry weight may be: 1-10 g/100 g for a total fat amount, 0-4 g/100g saturated fatty acids, 0-5 g/100 g monounsaturated fatty acids and 0-8 g/100 g polyunsaturated fatty acids.

The food product may comprise a hydrocolloid, optionally xanthan gum, locust bean gum, carrageenan, sodium alginate, starch and/or methylcellulose. This has been found to provide the food product as disclosed herein with a further enhanced structure and cohesiveness resembling the structure of meat-based products.

The pure fungi biomass of the present invention may have a Water Holding Capacity (WHC) within the range of from 2g to 4g of water bound per gram of the dry weight of the pure fungi biomass.

The pure fungi biomass may have a Water solubility Index (WSI) within the range of from 50% to 80%.

The pure fungi biomass may include an amount of dietary fibers is the within the range of from 5% to 25%, of the dry weight of the fungi biomass.

The food product may further comprise one or more ingredients selected from the following group: protein from soybean, pea, chickpea, wheat, rice, mung bean, potato, fava bean, lupin bean, egg or dairy; fat or oil from soybean, rapeseed oil, soybean oil, canola oil, coconut oil, sunflower oil or shea butter; binders and additives such as methylcellulose, xanthan gum, alginate, locust bean gum, agar-agar, gum arabic, egg white protein, sources of carbohydrates such as starch, wheat flour, potato flour, rice flour, oat flours, apple extract and sources of fiber such as pea, sugarcane, wheat, cellulose, oats and apple.

The food product may be in the form of a patty, nugget, burger, sausage, paste, chunk, fillet, extrudate, granules, cake, meat substitute, meat extender, jerky, fish-like product, seafood-like product, snack, beverage, dessert or baked good, or the like. The fungi biomass comprising fungi of a species belonging to the genus *Rhizopus* has been found by the present inventors to provide a non-animal based food product with a taste, smell and consistence with a high resemblance of meat-based food products but without the negative impact on the climate provided by meat-based products.

According to a second aspect, the present disclosure relates to a method for producing a food product, the method comprising the steps of:

a) cultivating fungi of a species belonging to the genus *Rhizopus* under aerobic submerged fermentation conditions using a closed fermentation vessel with liquid substrate media containing a carbon source originated from processed grain crops or glucose-, fructose- or lactose-containing substrates in a monomeric or oligomeric form to obtain a fungi biomass comprising fungi biomass fibers;

b) processing the fungi biomass obtained from step a) by heating to a temperature of from 50°C to 75°C for a period of time of from 5 minutes to 1 h;

c) separating the fungi biomass obtained from step b) from the liquid cultivation substrate, by pressing the fungi biomass in a second direction, thereby providing a pure fungi biomass having 50% or more, such as 70% or more, or 80% or more of the fungi biomass fibers aligned in planes extending in a first direction, the first direction being perpendicular to the second direction; and

d) incorporating the processed and pressed pure fungi biomass obtained in step c) in a food product.

The fungi biomass is either pressed in a single second direction or only in the second direction and in a third direction, the third direction being an opposed direction to the second direction and perpendicular to the first direction.

It has been found by the present inventors that when pressing the fungi biomass in a single direction, or from opposite directions, this results in a flow field, being perpendicular to the pressing direction, for the fungi biomass fibers that promotes alignment and creation of a lamellar structure, which has resemblances to meat when cut.

The pure fungi biomass from step c) may be mechanically treated to break the biomass into smaller pieces before incorporation of the fungi biomass into the food product in step d). For example, the pure fungi biomass may be chopped, mixed or sliced into smaller pieces.

The process for producing food-graded fungi biomass/mycoprotein is an aerobic submerged fermentation process. The group of fungi selected for this fermentation process are of the genus *Rhizopus.* This group of fungi has been found having a high growth rate and can be cultivated in continuous fermentation, meaning that the production can be made both highly quick and efficient. The process involves a single cultivation step and be made on an industrial scale, thus allowing a production of the food products disclosed herein to be an industrial scale production and it may be scaled to any desired size and volume.

The process that is considered a cultivation or aerobic fermentation process, i.e. the process carried out in step a), may take place in a stirred-tank reactor bioreactor, airlift reactor or bubble column reactor, where the liquid medium is agitated by aeration and/or stirring.

The cultivation in step a) may include stirring of the liquid substrate media and the fungi biomass at a rotation speed of from 100 rpm to 300 rpm.

The pressure step c) may include pressing with a pressing force within the range from 0.5 to 10 bar.

The method may prior to step c) and after step b) comprise a step b2) of incubating the heated fungi biomass in an aqueous solution having a salt concentration of from 0.5% to 10%, optionally the salt is NaCl.

The method prior to step c) and after step b) may comprise a step b2) of incubating the heated fungi biomass in an aqueous solution having a pH within the range of from 6 and 10.

The process related to the harvesting and post-fermentation treatment of the fungi biomass, includes a heat-treatment step, according to step b) for inactivation of the fungi biomass, a filtering and/or centrifugation step to separate the solid biomass from the liquid substrate and thereby obtaining a pure fungi biomass which is not possible, or at least very difficult, in a so-called solid-state fermentation process. The process may additionally include a washing step.

The pure fungi biomass obtained in step c) may have a water content within the range of from 25% and 80%, optionally within the range of from 50% and 80%, optionally the pure fungi biomass obtained in step c) have a water content within the range of from 60% and 80%.

The process for producing food-graded fungi biomass/mycoprotein as disclosed herein provides a food product which is rich in essential amino acids, suitable for human consumption, free of yeast protein and rich in dietary fiber.

The process disclosed herein is suitable for industrial scale production (> 10 m$^3$ reactor volume) of food products and can be scaled to practically any desired size and volume.

The food product may be mixed or homogenized in the presence of any one or more of the ingredients selected from the following group: protein from soybean, pea, chickpea, wheat, rice, mung bean, potato, fava bean, lupin bean, egg or dairy; fat or oil from soybean, rapeseed oil, soybean oil, canola oil, coconut oil, sunflower oil or shea butter; binders and additives such as methylcellulose, xanthan gum, alginate, locust bean gum, agar-agar, gum arabic, egg white protein, , sources of carbohydrates such as starch, wheat flour, potato flour, rice flour, oat flour, apple extract and sources of fiber such as pea, sugarcane, wheat, cellulose, oats and apple.

The process may include a blending step with binders, protein powders, textured proteins, fresh proteins, flours, fats, gluten, egg.

The fungi biomass may be frozen at a temperature below -10 C° before being incorporated in the food product or the food product of step d) may be frozen at a temperature below -10C°.

The cultivation in step a) may take place at a temperature within the range of from 25 to 37°C.

In step a) a pH within the range of from 2.5 to 7.5 may be maintained for the duration of the fermentation.

The fungi biomass may be mixed with a hydrocolloid solution, optionally in an amount of from 0.01% to 5% of the total fungi biomass dry weight. The hydrocolloid solution may be added either during or after separation of the fungi biomass step in step c). This may provide the food product as disclosed herein with an enhanced structure and cohesiveness.

The carbon source may originate from a wheat flour based substrate, optionally a wheat flour based substrate with minerals. A carbon source being a wheat based substrate has been seen to provide a high yield of fungi biomass.

The amount of the wheat flour based substrate may be within the range of from 5 g/L to 50 g/L dry weight based on the liquid substrate media.

The process as disclosed herein may be for producing a food product comprising the fungi biomass/mycoprotein as the main ingredient, up to 100%, with or without the addition of other (plant-based) protein sources, fats, binders or flours. The resulting food products can be from the in the form of a patty, nugget, burger, sausage, paste, chunk, extrudate, granules, cake, meat substitute, meat extender, jerky, snack, beverage, dessert or baked good. The process may be a process for producing a food product comprising lower than 1% dry weight of fermentable substrate of the total amount of the dry weight of the fungi biomass in the food product, optionally within the range of from 0% to 0.5 % dry weight of the fermentable substrate of the total amount of the dry weight of the fungi biomass in the food product.

The process for producing food-graded fungi biomass in submerged, aerobic fermentation, where the concentration of biomass in the fermentation liquid may be between 0.5-50 g/L fungi biomass (dry weight).

The food product described may comprise of up to 95% fungi biomass of the genus Rhizopus. The amount of fungi biomass/mycoprotein, essential amino acids, fibers will vary according to the nature of the food product, product or dish prepared, but will contain 10-100% fungi biomass/mycoprotein (dry weight per dry weight of product).

The food product described can comprise, besides the fungi biomass/mycoprotein, ingredients from the following group: protein from soybean, pea, chickpea, wheat, rice, mung bean, potato, fava bean, lupin bean, egg or dairy; fat or oil from soybean, rapeseed oil, soybean oil, canola oil, coconut oil, sunflower oil or shea butter; binders such as methylcellulose, starch, xanthan gum, locust bean gum, agar-agar, gum arabic, pea fiber, egg protein, wheat flour, potato fiber, potato fiber, oat flour, oat fiber, wheat gluten.

The process is a cultivation or aerobic fermentation process in a closed, sterile vessel with media containing a single or several different carbon sources originating from processed grain crops or glucose-, fructose- or lactose-containing substrates in a monomeric or oligomeric form.

The heat treatment step is a process used for inactivating the biomass after fermentation, whereby the fungi biomass together with the liquid fermentation substrate, a diluted version of it, or the biomass submerged in water, may be heated up to a temperature of 50-75°C for a period of time between 5 min and 1 h.

The fungi biomass may be washed after heat treatment, whereby the fungi biomass is rinsed with tap water, distilled water or water containing 0-5% of salt (NaCl), with a water temperature of 6-15°C, for 3-60 min. After washing the washed and rinsed fungi biomass may be pressed either in combination with the separation step, or after it, to a moisture content of 25-80%.

Further processing of the fungi biomass may include steps for the mechanical homogenisation of biomass, mechanical chopping of biomass to a smaller particle size, mixing and incorporation of inredients mentioned above to obtain a food product, freezing the fungi biomass and/or the food product containing fungi biomass to below -10°C.

The fungi biomass may subsequent to any one of steps c), d) be exposed to UV-B light, such as of 200-400 nm wavelength, and during a period of time of about 1 minute to 60 minutes. To expose the fungi biomass to UV-B light was found to increase the vitamin D2 levels to between 10 and 100 000 μg/kg and thus further enhancing the nutritional value of the food product.

The edible fungi biomass may be formed into a dried powder, whereby the fungi biomass is dried in a hot-air or convection oven at temperatures between 45°C and 105°C for 10-48 hours, and then milled in a ball mill, knife mill or coffee grinder to a fine powder.

According to a third aspect, a use of a pure fungi biomass for preparing a food product is disclosed herein. The pure fungi biomass comprises fermented fungi of a species belonging to the genus *Rhizopus* and the food product is in the form of a patty, nugget, burger, sausage, paste, chunk, fillet, extrudate, granules, cake, meat substitute, meat extender, jerky, fish-like product, seafood-like product, snack, beverage, dessert or baked good.

The pure fungi biomass and the food product may be a fungi biomass and a food product, respectively, as disclosed according to the first aspect. And the pure fungi biomass and the food product may be produced according to the second aspect.

DESCRIPTION OF FIGURES

Figure 1 shows images obtained through stereomicroscopy indicating alignment of fungal mycelium according to a structure having aligned fungi biomass fibers compared to commercial mycoprotein which does not present this structure.

Figure 2 shows a graph illustrating values of Hardness from a texture profile analysis (TPA) test of balls formed with fungi biomass mixed with different classes of ingredients.

Figure 3 shows a graph illustrating Compression Work from a texture profile analysis (TPA) test of balls formed with fungi biomass mixed with different classes of ingredients.

Figure 4 illustrates the results of a comparison with a meat-ball style food product according to the present disclosure and six other non-animal based meatball-style products and one animal-based meatball product.

Figure 5 illustrates the results of a comparison between a pure fungi biomass according to the present disclosure and other protein sources with respect to their respective water holding capacity (WHC).

Figure 6 illustrates the results of a comparison between a pure fungi biomass according to the present disclosure and other protein sources with respect to their respective water solubility index (WSI).

Figure 7 illustrates the results of a comparison between a pure fungi biomass according to the present disclosure and other protein sources with respect to their respective oil holding capacity (OHC).

Figure 8 illustrates the correlation between propeller stirring speed of a stirred tank bioreactor of 30L size and the Toughness of the biomass obtained, measured through a Knife Blade test on a texture analyzer.

Figure 9 shows three graphs illustrating parameters obtained from a Knife Blade test performed on fungi biomass samples which were grown in liquid bioreactors with two different type of blades: a high-shear mixing and a low-shear mixing blades.

Figure 10 shows a graph illustrating the values of toughness of a Knife Blade test performed on fungi biomass samples which were subjected to different concentrations of NaCl and then pressed, and compares them to meat samples and commercial mycoprotein.

Figure 11 shows a graph illustrating the values of toughness of a Knife Blade test performed on fungi biomass samples which were subjected to different pH values and then pressed, and compares them to meat samples and commercial mycoprotein.

Figure 12 shows the water content of different fungi biomass samples that were incubated with different concentrations of NaCl, at different pH values, or pressed at different pressures.

DETAILED DESCRIPTION

The present disclosure relates to the process for producing edible, food-grade fungi biomass/mycoprotein through a fermentation process on carbohydrate substrates, and the application of the resulting pure fungi biomass in a food product for human consumption. The filamentous fungi used in the invention are cultivated in liquid media under aerobic conditions and belong to the genus Rhizopus. The food products containing this fungi biomass are in the area of meat replacements, meat extenders, protein-enriched food products, fiber-enriched food products, or novel mycoprotein-containing food products.

To achieve the texture and taste profile acceptable and desired for these types of products, the biomass can be processed using the following described methods. After harvesting from the fermentation, the biomass needs to be heat treated at 50-70°C for 5-60 min, and after that a washing step may be applied to remove off-tastes. A pressing step may also be applied for dewatering after this treatment. For this pressing step, to create a good meat-like structure, the fungal mycelia should be pressed from one single direction, and allowed to expand in the direction perpendicular to the pressing direction. This creates a flow field for fibers that promotes alignment and creation of a lamellar structure, which has resemblances to meat when cut. In combination to this, the equipment may allow for the creation of large press cakes, which are beneficial for the creation of larger meat-like chunks, which have added value as opposed to small mince-like pieces. To create the combination of the pressing effect and obtaining large press cakes, the following industrial equipments can be used as examples: an hydropress, a piston press, a belt press, a cold-press or similar systems. Other common dewatering equipments such as conventional filter presses and membrane filter presses will create forces in multiple directions while the fungi biomass is concentrated and dewatered, and therefore not create the desired alignment, while other equipments like decanters and centrifuges will not allow the obtention of large filter cakes and might not also exert a pressing force in a single direction.

The pressing step affects significantly the biomass texture so that biomass conditions before and during pressing step influence the outcome. Salts and other solutes causing osmotic pressure such as but not limited to NaCl can be added to the biomass-containing solution before pressing in concentrations of, for example, 0.1% to 10%, which has an effect of decreasing the biomass toughness values when measured with a knife blade shear test in a texture analyzer. Toughness of the pressed biomass, as well as dry matter content of the press cake is also influenced by adjusted pH of the biomass-containing solution before pressing. Increase in pH is correlated to decrease in toughness, and pressing should occur between pH 6 and 10 for the better effects of creating a product with meat-like toughness values.

Fungi biomass

The present disclosure relates to pure fungi biomass produced by a fermentation/cultivation process from a starter-culture/pre-culture. The strain of filamentous fungi belong to the genus of Rhizopus, such as *Rhizopus oryzae, Rhizopus microsporus var. oligosporus* (also called *Rhizopus oligosporus*), *Rhizopus arrhizus, Rhizopus delemar, or Rhizopus stolonifer.*

The pure fungi biomass is an edible product for use in a human food product, either alone or in combination with other ingredients.

The pure fungi biomass is rich in protein with an unusually favourable amino acid composition, low carbohydrate content and low fat content. The percentage of protein is in the range of 25 to 70%. The percentage of essential amino acids is 30-70% of the total amino acid content. The percentage of dietary fiber is in the range of 1 to 10%. The values for fatty acids in dry weight are: 0.1-10 g/100 g for total fat, 0-4 g/100g saturated fatty acids. 0-5 g/100 g monounsaturated fatty acids and 0-8 g/100 g polyunsaturated fatty acids.

**Experimental section**

*1.1 Microorganism*

The zygomycete fungi *Rhizopus oryzae, Rhizopus oligosporus, Rhizopus arrhizus, Rhizopus delemar,* and *Rhizopus stolonifer* were used throughout these tests.

The strains were maintained on potato dextrose agar (PDA) plates containing (in g/L): agar 15, dextrose 20, potato extract 4. New plates were prepared by incubating for 4-5 days at 35°C and then storing them in the fridge (4°C). The plates were renewed every 2 months from a 25% glycerol stock kept at -80°C.

*1.2 Inoculum preparation*

For preparation of spore suspension, a plate was flooded with 10-20 mL of sterile water and spores scraped off the surface with a disposable, sterile spreader. Spores were counted in a hemocytometer under a light microscope, and used directly as inoculum for plates and liquid cultivations.

*1.3 Cultivation in shake flasks*

*R. oryzae, R. oligosporus, R. arrhizus, R. delemar, and R. stolonifer* were cultivated in Erlenmeyer flasks (volumes 100-2000 mL) with or without baffles, filled with liquid growth medium to a maximum of 20% of the total flask volume. Growth media contained either glucose or sucrose as the carbon source in the range of 10-40 g/L.

Additional media components: nitrogen source in the form of ammonium, as well as potassium, magnesium and calcium in the form of phosphates, sulphates or chlorides.

1 mL of spore suspension (10 ^7 spores/mL) per 100 mL of growth media was added to each flask, followed by incubation at 30-35°C for 18-24h under shaking (100-150 rpm).

### 1.4 Cultivation in bioreactor

To 20 L growth media in a 30 L stirred-tank bioreactor, 1-3 L of preculture from shake flasks were added, as well as 10-50 mL of spore suspension. The pH was adjusted to 5-6 with 5M NaOH. Sterilisation of the liquid in the bioreactor was done by heating up the liquid with steam (via the bioreactor's double jacket) to 121°C and 1 bar overpressure for 20 min.

Batch cultivation was carried out for 24h, at 30-35°C, stirring at 150 rpm, under aeration, and the pH was kept between 5.0 and 6.0. Semi-continuous cultivation was carried out by harvesting part of the biomass and growth media after 18-24h, and filling up to 20L again with fresh, sterile growth media.

A fungal biomass yield of between 0.2 and 0.3 g pure biomass/g substrate could be reached at the end of the fermentation (24h).

### 1.5 Protein content of dry fungal biomass

Fungal biomass harvested from shake flask and bioreactor cultivations was dried at 65-105°C for 24-48h until no change in mass occurred and all water had been removed. The dried biomass was milled in a TissueLyser ballmill for 30 sec to obtain a fine powder. The elemental composition of the powder was analysed in an Elemental Analyser to obtain the percentages of nitrogen, hydrogen, sulphur and oxygen. To estimate the protein content of the fungal biomass, the nitrogen content of the dried biomass was multiplied with the factor 6.25.

### 1.6 Nutrient content of Rhizopus fungal biomass

The nutritional composition of the Rhizopus biomass was analysed in detail by an external accredited laboratory (ALS Scandinavia AB). One representative example of the nutritional composition of the dry biomass showed the following values (per 100 g): 340 kcal (or 1400 kJ), 60.29 g protein, 3.74 g carbohydrates, 5.97 g fat, 12.30 g fiber. One representative example of the nutritional composition of the wet biomass showed the following values (per 100 g): 85 kcal (or 350 kJ), 15.07 g protein, 0.94 g carbohydrates, 1.49 g fat (of which 0.34 g saturated fat, 0.40 g monounsaturated, 0.68 g polyunsaturated fat), 3.08 g fiber.

One representative example of the amino acid content (% of total protein) of the fungal biomass of Rhizopus is summarised in Table 1 below, with essential amino acids being marked with an asterisk.

| | |
|---|---|
| Alanine | 10.16% |
| Arginine | 3.39% |
| Asp. Acid | 5.98% |
| Cystine | 1.39% |
| Glut. Acid | 8.37% |
| Glycine | 7.17% |
| Histidine* | 3.59% |
| Isoleucine* | 7.97% |

| | |
|---|---|
| Leucine* | 9.36% |
| Lysine* | 8.96% |
| Methionine* | 2.99% |
| Phenlyalanine* | 5.78% |
| Proline | 2.39% |
| Serine | 2.59% |
| Threonine* | 6.18% |
| Tryptophan* | 0.80% |
| Tyrosine | 3.78% |
| Valine* | 9.16% |
| Essential amino acids | 54.78% |
| BCAA | 26.49% |

*Table 1: Content of amino acids (in % of total protein) in Rhizopus biomass.*

Regarding micronutrients, the following values were obtained for one representative measurement of the dry Rhizopus biomass (per 100 g): 509.1 mg calcium, 1004.5 mg potassium, 116.5 mg magnesium, 148.6 mg sodium, 238.5 mg sulfur, 2550.2 mg phosphorus, 10.8 mg iron, 15.5 mg zinc, 5.2 mg copper, <0.2 ug vitamin B12, 1.0 ug vitamin B6, 1.5 ug vitamin D2 and <12.5 ug vitamin D3.

**Experiment 2: Cultivation on synthetic media in 300 L bioreactor**

*2.1 Process parameters*

Cultivations on sugar substrate were scaled up to a 300 L bioreactor (manufactured by Belach Bioteknik) with a working volume of 250 L. The process parameters were the following: 25-45 L/min constant aeration through an arrangement of porous air spargers, 30-35°C, stirring with a single Rushton impeller, pH 5-6 (adjusted with 5M NaOH). The cultivation media had the same composition as described in 1.3 with sucrose as the main carbon source.

For batch cultivation, all biomass was harvested after 24h. For semi-continuous cultivation, an initial batch cultivation was carried out for 24h, followed by harvesting of 50-80% of the fermentation liquid and filling up with fresh, sterile growth media to 250 L for another round of 18-24h fermentation.

*2.2 Heat treatment regimes*

Heat treatment of biomass to inactivate the fungus was conducted at 50-70°C for 5-60 min. The best results were achieved by heat-treating the biomass at 55-65°C for 10-20 min. Several different methods were tried, including heating up the biomass together with the fermentation liquid to 60°C in the bioreactor, or immersing the harvested biomass in 60-65°C hot water. The success of heat inactivation was checked by placing heat-treated biomass on PDA plates and incubating at 35°C for 24h. Sporulation showed an insufficient heat treatment.

The best results were achieved with immersing the harvested and filtered biomass into 65°C water for 10 min while stirring. No sporulation was detected after this treatment, and the biomass had a pleasant texture and appearance without appearing too cooked.

*2.3 Fed-batch/semi-continuous fermentation mode*

The concentration of dry biomass obtained during the semi-continuous process was 3.12 g/L, with a resulting productivity (g/L*h) of 0.134 dry biomass.

**Experiment 3: Production of a meat/chicken-like product containing *Rhizopus* biomass**

*3.1 Fermentation process parameters*

The process parameters were the same as described in Experiment 2.

*3.2 Treatment of biomass after fermentation*

Upon harvesting, the biomass was subjected to a heat treatment step. The biomass was washed and pressed in a single direction in order to remove water and orient the fibres in a single plane. For minimally treated samples, the pressed biomass with a water content of 70-80°C, was broken into small pieces. The biomass had a fibrous texture, no off-flavour or off-taste and a beige colour.

Prior to pressing, one sample contained hydrocolloids integrated into the biomass network by mixing the biomass with a hydrocolloid solution of 1 to 5%. The resulting press cake with or without integrated hydrocolloids was sliced into chunks ranging from 1 cm to 10 cm in size. Said chunks were then further reduced in size by processing in a blender or hacking machine by themselves or in the presence of a hydrocolloid solution added during the size reduction step.

The fungal biomass treated with hydrocolloid solution, when grinded to small particles, forms a mince-like form that has binding properties among the biomass particles, which was not a strong trait of the raw biomass without addition of said hydrocolloid. The presence of hydrocolloid solution with the biomass also resulted in an increase of water retention capacity when the mix was subjected to a freeze-thaw cycle. Within 24h at 4°C after thawing the samples, the biomass sample alone would lose up to 24% of its weight as water released. The addition of xanthan gum after the biomass pressing step decreased this to an 8% weight loss, while addition to the biomass before the pressing step decreased it to a 5% loss.

*3.3 Preparation of food product functional formulations*

The biomass that was mixed with different solutions of hydrocolloids was successfully used in the forming of balls and patties such as burgers. The structure and cohesiveness of the ball/patty were improved when solutions of the hydrocolloids xanthan gum, locust bean gum, sodium alginate, potato starch or methylcellulose were used, compared to the same structure formed with the biomass without use of any additions.

The mixing of biomass at 60-85% water content with sodium alginate and starch at 2% (w/w) each, and subsequent treatment with heat in a wet environment (steaming or boiling), led to the gelation of the obtained structure, making it suitable for use in sliceable meat alternative food products.

*3.4. Shelf life analysis*

The microbiological activity of the biomass and preparations of such was analyzed after treatment and after conservation at -18°C for 6 months. No activity was detected either in the fresh biomass post-treatment of conserved in the freezer for 6 months.

In another experiment, formed balls using the fungi biomass in the presence of hydrocolloids were conserved in vacuum at 4°C for 8 days. After reopening the vacuum packages, samples with and without hydrocolloids were equally well preserved. The taste, flavour, texture and smell profiles were similar to recently defrosted or freshly produced samples.

*3.5 Comparison of fiber texture by stereomicroscopy*

Samples of the pressed biomass or of a commercial mycoprotein chicken-like product were generated by cross-cutting surfaces with a razor blade while frozen. These samples were then examined with a Zeiss SteREO Discovery.V8 stereomicroscope equipped with Achromat S 0.5x objective (Carl Zeiss Microlmaging GmbH, Göttingen, Germany) and imaged using an Olympus DP-25 single chip colour CCD camera (Olympus Life Science Europa GmbH, Hamburg, Germany) and the Cell^P imaging software (Olympus).

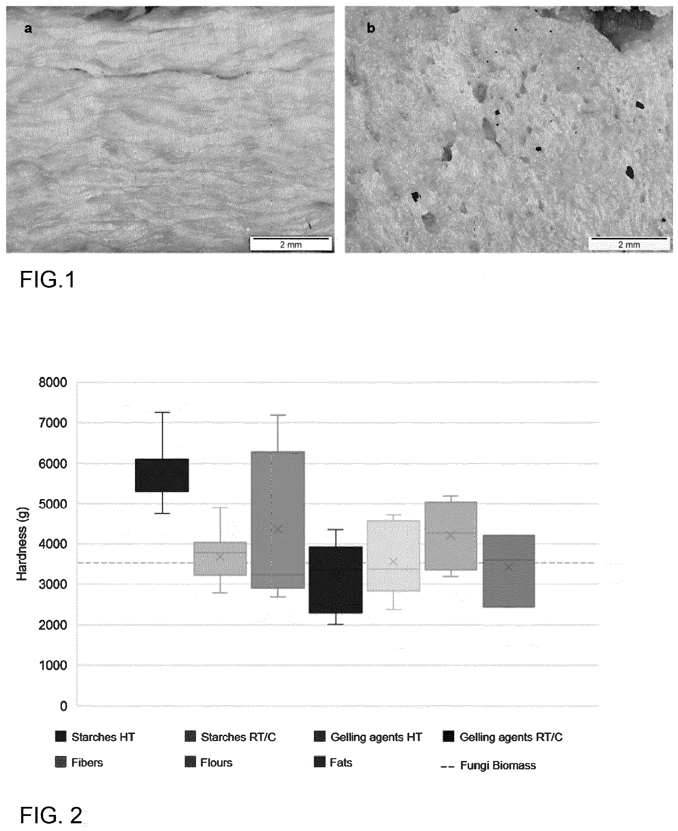

The obtained images in Figure 1 show that the fungal mycelium from pressed samples is substantially aligned forming a lamellar structure, which is not present in the existing commercial mycoprotein product. This structure creates a much more appealing product as a meat replacement compared to the existing market alternatives.

Samples were pressed in a hydropress system with radial pressure, in a belt press system, and in a hydraulic piston-based system that apply the same concepts of single direction force applied with room for perpendicular expansion. The same product effect was observed as in Figure 1.

**Experiment 4: Production of a final product ("meatball") containing *Rhizopus* biomass**

*4.1 Preparation of product from fungi biomass*

Upon harvesting, the biomass was subjected to a heat treatment step. The biomass was washed and pressed in a single direction in order to remove water and orient the fibres in a single plane. The biomass was then frozen under vacuum conditions and thawed after a week. The biomass was cut down to pieces from 0.5 to 2 cm and blended together with selected culinary ingredients from a list of ingredients with synergistic effects for the product texture, such as oat flour, potato starch, almond flour, rice flour, wheat flour, corn starch and bread crumbs. To this was also added flavour ingredients such as spices, salt, sugar and flavour enhancers. To this it was also added hydrocolloids such as methylcellulose, sodium alginate or carrageenan. The mix was blended for up to 15 minutes and balls were formed with the resulting mass. The balls were heat treated in an oven at 140°C for 40 min and deep fried at 180°C for 2 min.

*4.2 Sample preparation of mycoprotein mixed with different ingredients for Texture Profile Analysis*

Samples of pure frozen biomass with water content between 70% - 75% were defrosted and ground with a single-blade enterprise cutting system using a hole diameter of 8 mm. The ground biomass was then mixed with different ingredients composing of different starches, gelling agents, fibers, flours, fats and proteins in a concentration of 5% for a sample in the shape of a ball with 15g weight. In the case of gelling agents, a 5% solution was prepared according to manufacturer's indications and 5% was added as an ingredient. Ingredients were also heat-treated by incubating in an oven for 125°C for 15 min or cold-treated by placing at -4°C for 2h.

**4.3 Texture Profile Analysis (TPA)**

Binding properties of these ingredients were analyzed through a standard Texture Profile Analysis using a Stable Microsystems TA.TX Plus-C equipped with a P/100 Stainless Steel Compression Platten with a diameter of 100 mm and an acquisition rate of 500 PPS. The plate was set to compress the samples by 60% of the sample size using a trigger force of 20 g in a 2-cycle analysis at a test speed of 1 mm/sec. The deformation curve of the sample was obtained, from which the parameters Force 1, Force2, Area FT1:2, Time-diff 1:2, AreaFT1:3, AreaFT2:3, AreaFT3:4 (negative), AreaFT4:6, and Time-diff4:5, according to the manufacturer's protocol. From these parameters, the following parameters were calculated:
Hardness = Force2 (peak force of the first compression of the product) Cohesiveness = AreaFT4:6 / AreaFT1:3 (area of second deformation relative to area of the first deformation)

$$\text{Gumminess} = \text{Hardness} \times \text{Cohesiveness}$$

Springiness = Time-diff4:5 / Time-diff41:2 (percentage of product height that is regained after first deformation)

$$\text{Chewiness} = \text{Gumminess} \times \text{Springiness}$$

Resilience = AreaFT2:3 / AreaFT1:2 (area of the second half of the first deformation relative to area of the first half)
Adhesion = AreaFT3:4 (pulling strength during retraction from sample)

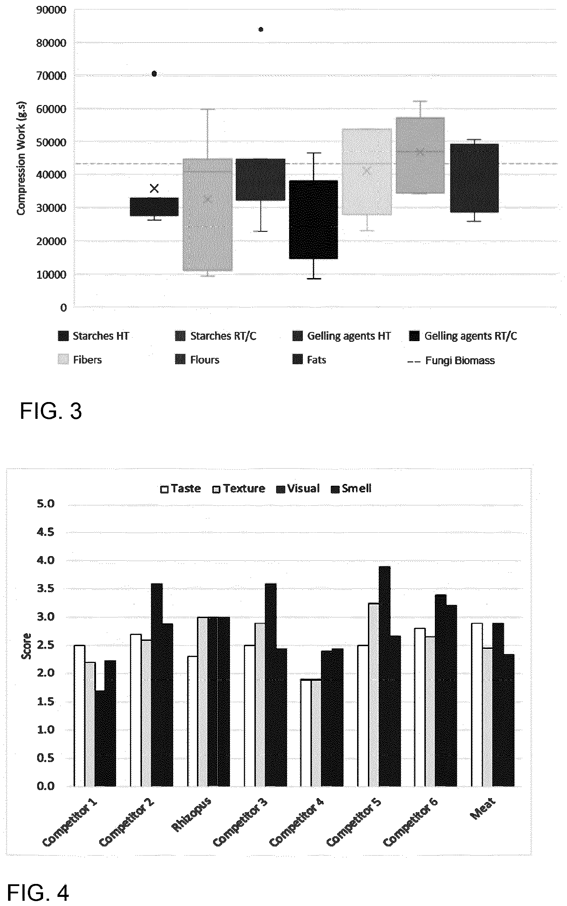

Mixing with the different ingredients changed all parameters measured by the TPA in the fungi biomass samples in significant ways, from -95% to up to 500% as seen in Figure 2 and Figure 3.

Fig. 2 shows a graph illustrating values of Hardness from a texture profile analysis (TPA) test of balls formed with fungi biomass mixed with different classes of ingredients. Each box represents the variation of values within a single class of ingredients. For some classes, different boxes were created for each temperature treatment (HT: High-temperature, RT/C: Room temperature/cold).

Fig. 3 shows a graph illustrating values of the Compression Work of the first peak from a texture profile analysis (TPA) test of balls formed with fungi biomass mixed with different classes of ingredients. Each box represents the variation of values within a single class of ingredients. For some classes, different boxes were created for each temperature treatment (HT: High-temperature, RT/C: Room temperature/cold).

This shows the compatibility of the fungi biomass with many ingredients used in the food industry to achieve a variety of textures.

### 4.2 Sensory analysis

A small sensory test panel evaluated 7 competitor plant-based ball products, as well as a meat-based ball product, regarding visual appearance, smell, texture and taste. The testers classified the balls made from Rhizopus fungi biomass to be of identical (non-statistically significant difference) quality to 4 of the existing plant-based commercial alternatives and superior to 3 others (Figure 1). The result of the sensory test is illustrated in Figure 4.

### 4.3 Storage conditions

The products created with the fungi biomass were frozen and conserved at -18°C. Packaging the products in vacuum conditions eliminated any gain of off-flavours and unpleasant taste over time and preserves the product longer due to elimination of oxygen.

## Experiment 5: Production of a dry biomass-containing food ingredient

### 5.1 Preparation of dried fungi biomass

Biomass obtained from the fermentation process was harvested, filtered and pressed to remove water. The pressed biomass was cut into small pieces and dried at 85-105°C over the course of 16-24h to remove water. The resulting dry mass was grinded in a ball mill to obtain a fine powder.

### 5.2 Water holding capacity and water solubility index

The method used for measuring water holding capacity (WHC) and water solubility index (WSI) was adapted from Miedziandka et al, with modifications (Miedzianka et al., 2014; https://doi.org/10.1016/j.foodchem.2014.03.054). 0.1 g of dry fungal biomass powder (dried at 45°C until all water was removed and milled in a ball mill) was added to each tube and the exact biomass weight was noted down. 10 mL of MilliQ-water was added, creating a 1% suspension (w/v). The samples were then stirred for 20 s every 10 min for one hour using a laboratory vortex mixer on full speed. The samples were centrifuged (4000g, 25 min, room temperature). The supernatant was discarded and added to pre-weighed beakers and left to dry overnight at 85°C. The pellets left in the centrifuge tubes were incubated at 50°C for 25 min before weighted.

The values of the dried pellets, supernatants and volumes were used for calculating the WHC and WSI according to the formulae used in Miedziandka et al. 2014:

Initial weight of sample = A
Final dried weight of remaining pellet = B
Final dried weight of supernatant = C

$$WHC = \frac{B - A}{A}$$

$$WSI = \frac{A - C}{A}$$

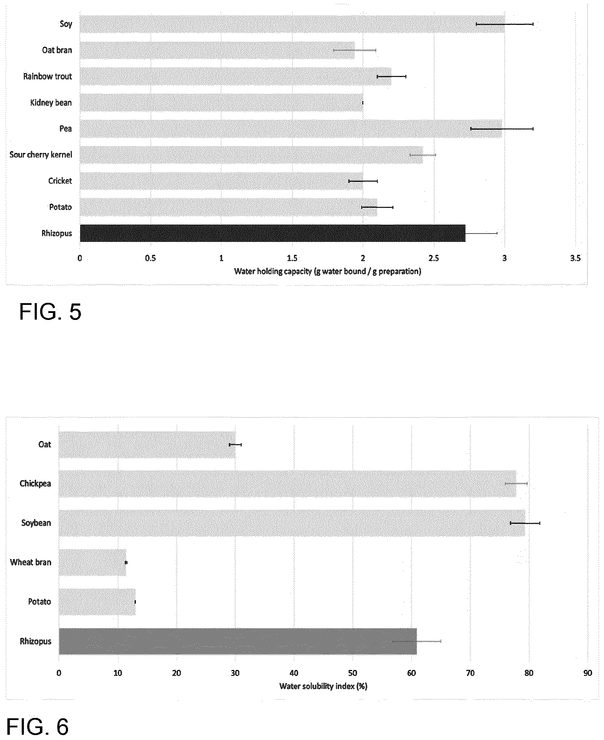

Comparisons were made with commonly used protein sources such as pea and soy protein, Rhizopus biomass had a comparable WHC as illustrated in Figure 5.

As to the WSI and as illustrated in Figure 6, Rhizopus biomass has a comparable WSI to the commonly used protein from soybean and chickpea.

### 5.3 Oil holding capacity

The method used for measuring oil holding capacity (OHC) was adapted from Miedziandka et al., with modifications (Miedzianka et al., 2014; https://doi.org/10.1016/j.foodchem.2014.03.054).. 500 mg of dry fungal biomass powder (dried at 45°C until all water was removed and milled in a ball mill) was added to 15 mL centrifuge tubes (the exact weight was noted down), followed by addition of 5 mL corn oil. The samples were mixed for 30s using a laboratory vortex mixer on full speed. The samples were then centrifuged (2000g, 30 min, RT), and the volume of oil separated after centrifugation was determined using a measure cylinder. These values were used to determine the OHC according to the formula used in Miedziandka et al. 2014:

D = Initial volume of oil
E = Final volume of oil separated
F = Weight of sample added

$$OHC = \frac{D - E}{F}$$

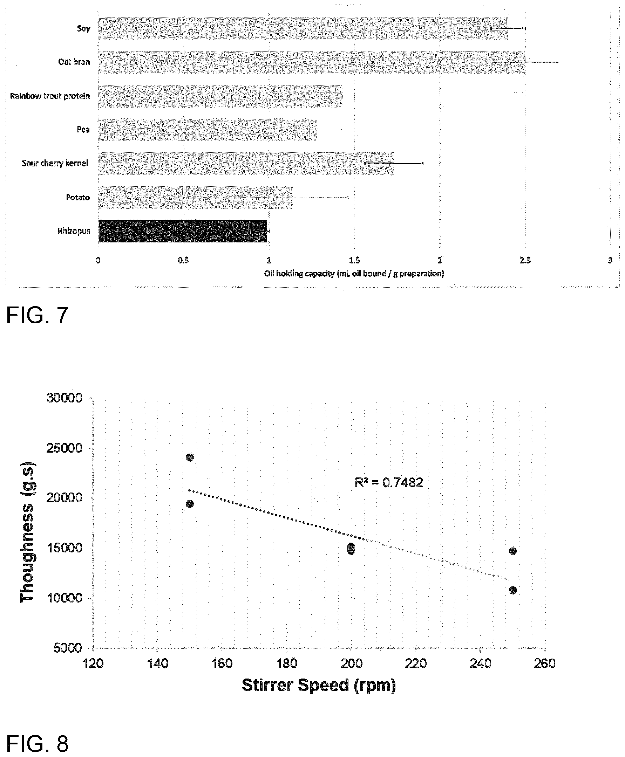

As illustrated in Figure 7 and compared to commonly used protein, Rhizopus fungi biomass has a fairly low OHC, similar to potato and pea protein.

**Experiment 6: Production of edible biomass with increased vitamin D content**

*6.1 Preparation of fungal biomass*

Upon harvesting, the biomass was subjected to a heat treatment step. The biomass was washed and pressed to remove water. The pressed biomass was cut into small pieces, vacuum-packaged and frozen.

*6.2 UV treatment*

For the UV treatment, half-thawed Rhizopus biomass was spread in a 2 cm layer onto the surface of a UVP Transilluminator PLUS (Jena Analytik) and exposed to UV-B light (302 nm, 25 W) at setting "mild". The biomass pieces were covered with aluminium foil and exposed for 10 min and mixed to ensure even exposure. This was repated every 10 min for 45 min in total. As control, an unexposed sample was included in subsequent analysis. Samples were frozen again and sent to an external lab for analysis of vitamin D2 and D3 content.

*6.3 Vitamin D content results*

The UV-exposed samples and reference (non-exposed samples) were analysed for Vitamin D2 and Vitamin D3 content. The UV-treated samples reported a 1878-fold improvement in Vitamin D2 levels compared to the non-exposed samples. (Table 2) The protein content of the biomass was not affected by the UV treatment.

*Table 2: Vitamin D2 and D3 contents of UV-exposed Rhizopus biomass.*

| Sample | Vitamin $D_2$ ($\mu$g/kg) | Vitamin $D_3$($\mu$g/kg) |
|---|---|---|
| Reference | 32.3 | <12.5 |
| UV-treated samples | 60 100 | <12.5 |

*Experiment 7: Varying shear rate and profile during liquid fermentation*

*Evaluating effect of different shear rates in bioreactor mixing*

Aerated bioreactor fermentations were carried out as described in Experiment 1, section 1.4. Fermentation experiments were performed at varying stirrer rotation speeds (150 rpm, 200 rpm and 250 rpm), in which increased stirring speeds created higher shear rates in the fermentation broth. The fermentation was carried out multiple times with varying rotation speeds.

Samples were then harvested and pressed in a hydropress at a pressure of 2.5 bar to remove water, promote fiber alignment and create a solid material with less than 80% water content.

*Texture analysis using a Knife Blade method*

Samples of solid fungi biomass were prepared as a cuboid shape of 20 mm x 10 mm x 5 mm (length x width x height) for texture analysis. Texture analysis was carried using a Stable Microsystems TA.TX Plus-C equipped with a Knife Blade (70 mm width x 3 mm thick, 45°-chisel end) and guillotine block. The sample was placed in the centre of the guillotine block and cut with the knife blade starting at a position of 20 mm and a descending speed of 1 mm/s until trigger force of 20g is sensed, which in turn the plot was acquired as the blade moved for 20 mm at 2 mm/s. A curve plot was obtained showing measured Force x Time, and the parameter of Toughness was defined as the total area below the curve in g s.

Toughness values varied between 25000 g.s and 10000 g.s and a correlation between higher stirrer speeds (higher shear rate) and lower toughness values was found with an R2 = 0.748. Results can be seen in Figure 8.

*Evaluating effect of different mixing blade types*

Fungi biomass was grown as described in experiment 2 in a 300L vessel with a 3-blade propeller stirring system. A second system was also setup with the same conditions but using a Rushton turbine as stirring system. 3-blade propellers provide a low-shear radial mixing while Rushton turbines provide high shear radial mixing. Samples were harvested from the two bioreactors and processed in the same conditions as described in experiments 2 and 3. Samples from the high shear fermentation (Rushton turbine) had a profile observed by a panel of 4 people described as more "grainy", "crumbly" and having a "shorter bite", and less meaty than the low shear (3-blade propeller) fermentation samples.

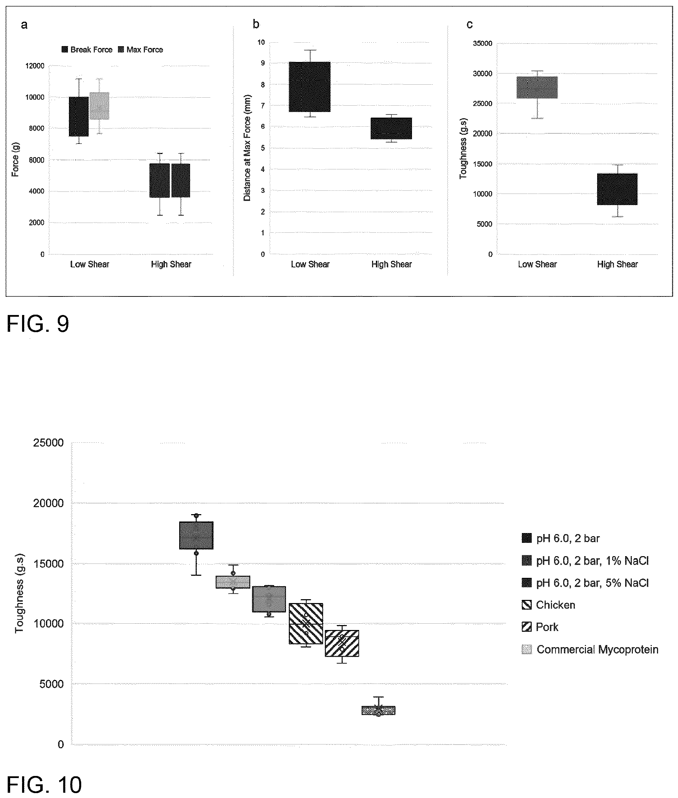

Texture analysis using the Knife Blade method described above was performed on samples derived from the two different bioreactor systems. The plots of force x blade distance were analyzed and the profile from the two samples were significantly different. The force profile of high shear samples was often a single peak, indicating a brittle material with one single breaking point, while the low-shear samples had often multiple peaks, indicating several pressure points over the blade penetration. The following parameters were measured: "Break force" indicates the force value of the first peak observed in the plot, "Max force" indicated the force value of the highest peak in the plot, "Distance to max force" indicates the blade travel distance from trigger point (trigger of 20g force) at which Max force is measured, "Toughness" indicates the total positive area of the plot. Results are shown in Figure 9. Figure 9, panel a, shows that for many points in the low shear sample the break force is lower than the max force, while in the high shear these are equal, and the force values are much lower for the high shear samples. This indicated a brittleness and easier breaking in the high shear samples and a longer bite with several pressure peaks in the low shear samples. Figure 9 panel b shows how the max force distance is much lower for the high hear samples, meaning that high shear samples not only break easier, they break sooner. The toughness values in panel c are also much higher or lower-shear samples, meaning there is a much higher chewiness when biting for low shear samples. These results indicate lower shear fermentation such as by using propeller blades is more suitable to produce meat-like products since the bite profile is much more favourable for these products.

**Experiment 8: Use of different pressing conditions with salinity and pH**

Fungi biomass was produced as explained in experiment 2. Prior to the pressing step and after the washing step, the biomass was incubated for 30 minutes in just water or water 1% or 5% NaCl concentrations, or pH values of 3.0, 6.0, 9.0 or 11.7. The pH was adjusted by addition of NaOH or HCl. The incubated biomass was then placed in a hydropress system which applied a directional radial pressure so that the samples were pressed in a single plane and could flow/expand in a perpendicular direction to the force applied. The press was allowed to reach 0.5, 1.0 or 2.0 bar and held at the intended pressure for 5 minutes.

In parallel, to compare with the texture of meat, pieces of chicken breast (chicken) and pork chop (pork) were boiled in boiling water for 25 minutes and then allowed to cool down to room temperature. To compare with the texture of another mycoprotein "chicken-like bites" available in the market, a sample of "Quorn bites" (Marlow foods) was bought from a retailer and thawed to room temperature. Samples of fungi biomass, meat and commercial mycoprotein samples were then prepared and analysed by texture analysis using the Knife Blade method as described in Experiment 7.

Figure 10 illustrates toughness values of three different fungi biomass samples according to the present invention, one being incubated in water, one being incubated in 1% NaCl and one being incubated in 5 % NaCl prior to a respective pressing step at 2.0 bar. The results shown in Figure 10 show that addition of 1% NaCl reduces the toughness of the sample significantly, while 5% NaCl produces a sample that has toughness values close to boiled chicken breast. All these samples were tougher than the commercial mycoprotein product. The effect of salt is thought to be related to variations of osmotic pressure in the cells that results in a different level of turgency pressure, therefore affecting product toughness.

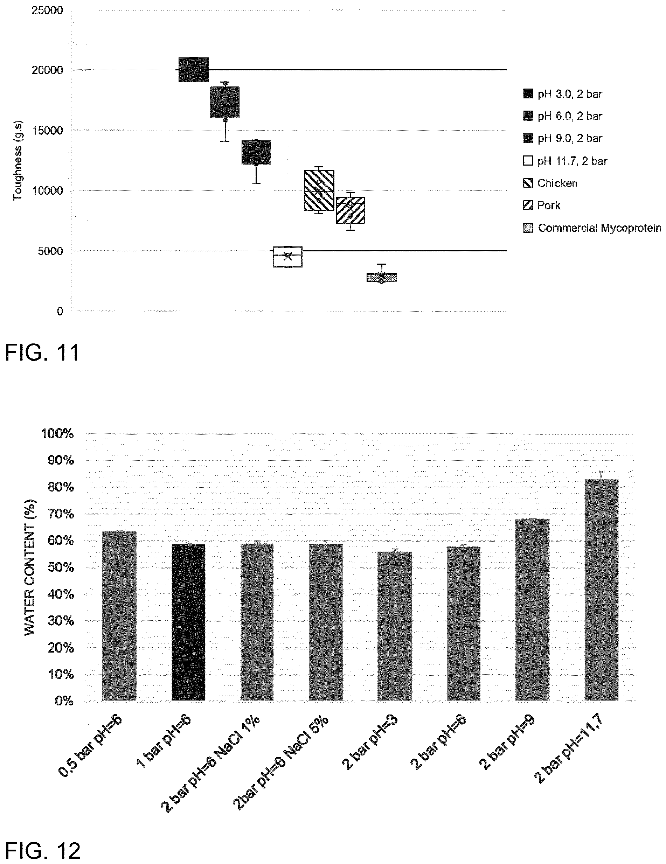

Figure 11 illustrates toughness values of three different fungi biomass samples according to the present invention pressed at 2.0 bar and with respective pH value of 3.0, 6.0 and 9.0 prior to pressing. The samples at different pH values also shown significant differences, in which high pH values is associated with lower toughness. Samples at pH 9.0 had a toughness closest to the meat samples.

The water content of all samples above was measured, which is shown in Figure 12. Samples at pH 9 and 11.7 showed much higher water content, and were also perceived as softer. This indicates changes in pH affect the water holding capacity of the mycelium and this can be used to retain more water, resulting in juicier products, but also having a softer texture. Increases in pH is therefore beneficial for meat-like characteristics such as increased juiciness and adequate toughness.

**Claims**

1. A food product comprising a pure fungi biomass, the pure fungi biomass comprising fungi of a species belonging to the genus *Rhizopus,* wherein the dry weight of the food product comprises within the range of from 10% to 100% of dry weight of the fungi biomass, the fungi biomass comprising fungi biomass fibers, wherein 50% or more, such as 70% or more, or 80% or more of the fungi biomass fibers are aligned in planes extending in a first direction.

2. The food product according to claim 1, wherein the dry weight of the food product comprises within the range of from 25% to 100% of dry weight of the pure fungi biomass.

3. The food product according to claim 1, wherein the dry weight of the food product comprises within the range of from 50% to 100% of dry weight of the pure fungi biomass.

4. The food product according to any one of claims 1 to 3, wherein the fungi are of the species *Rhizopus oryzae, Rhizopus microsporus var. oligosporus, Rhizopus arrhizus, Rhizopus delemar, or Rhizopus stolonifer.*

5. The food product according to any one of the preceding claim, wherein the amount of protein in the total pure fungi biomass (dry weight) is within the range of from 25% to 70%.

6. The food product according to any one of the preceding claim, wherein the amount of essential amino acids in the fungi biomass (dry weight) is within the range of from 30% to 70% of the total amino acid content.

7. The food product according to any one of claims 1 to 6, wherein the food product further comprises hydrocolloid, optionally xanthan gum, locust bean gum, carrageenan, sodium alginate, starch or methylcellulose.

8. The food product according to any one of the preceding claims, wherein the food product further comprises one or more ingredients selected from the following group: protein from soybean, pea, chickpea, wheat, rice, mung bean, potato, fava bean, lupin bean, egg or dairy; fat or oil from soybean, rapeseed oil, soybean oil, canola oil, coconut oil, sunflower oil or shea butter; binders and additives such as methylcellulose, xanthan gum, alginate, locust bean gum, agar-agar, gum arabic, egg white protein, , sources of carbohydrates such as starch, wheat flour, potato flour, rice flour, oats, apple extract and sources of fiber such as pea, sugarcane, wheat, cellulose, oats and apple.

9. The food product according to any one of the preceding claims, wherein the food product is in the form of a patty, nugget, burger, sausage, paste, chunk, fillet, extrudate, granules, cake, meat substitute, meat extender, jerky, fish-like product, seafood-like product, snack, beverage, dessert or baked goods.

10. A method for producing a food product, the method comprising the steps of:

a) cultivating fungi of a species belonging to the genus *Rhizopus* under aerobic submerged fermentation conditions using a closed fermentation vessel with liquid substrate media containing a carbon source originated from processed grain crops or glucose-, fructose- or lactose-containing substrates in a monomeric or oligomeric form to obtain a fungi biomass comprising fungi biomass fibers,
b) processing the fungi biomass obtained from step a) by heating to a temperature of from 50°C to 75°C for a period of time of from 5 minutes to 1h,
c) separating the fungi biomass obtained from step b) from the liquid cultivation substrate by pressing the fungi biomass in a second direction thereby providing a pure fungi biomass having 50% or more, such as 70% or more, or 80% or more, of the fungi biomass fibers aligned in planes extending in a first direction, the first direction being perpendicular to the second direction, and
d) incorporating the processed and pressed pure fungi biomass obtained in step c) in a food product.

11. The method according to claim 10, wherein the pure fungi biomass from step c) is mechanically treated to break the biomass into smaller pieces before incorporation of the fungi biomass into the food product in step d).

12. The method according to claim 10 or 11, wherein the food product is homogenized in the presence of any one or more of the ingredients selected from the following group: protein from soybean, pea, chickpea, wheat, rice, mung bean, potato, fava bean, lupin bean, egg or dairy; fat or oil from soybean, rapeseed oil, soybean oil, canola oil, coconut oil, sunflower oil or shea butter; binders and additives such as methylcellulose, xanthan gum, alginate, locust bean gum, agar-agar, gum arabic, egg white protein, sources of carbohydrates such as starch, wheat flour, potato flour, rice flour, oats, apple extract and sources of fiber such as pea, sugarcane, wheat, cellulose, oats and apple.

13. The method according to any one of claims 10 to 12, wherein the pure fungi biomass is frozen at a temperature below -10 C° before being incorporated in the food product or wherein the food product of step d) is frozen at a temperature below -10C°.

14. The method according to any one of claims 10 to 13, wherein the cultivation in step a) takes place at 25 to 37°C.

15. The method according to any one of claims 10 to 14, wherein the cultivation in step a) includes stirring of the liquid substrate media and the fungi biomass at a rotation speed of from 100 rpm to 300 rpm.

16. The method according to any one of claims 10 to 15, wherein the pressure applied in step c) is within the range from 0.5 to 10 bar.

17. The method according to any one of claims 10 to 16, wherein the method prior to step c) and after step b) comprises a step b2) of incubating the heated fungi biomass in an aqueous solution having a salt concentration of from 0.5% to 10%, optionally the salt is NaCl.

18. The method according to any one of claims 10 to 16, wherein the method prior to step c) and after step b) comprises a step b2) of incubating the heated fungi biomass in water an aqueous solution having a pH within the range of from 6 and 10.

19. The method according to any one of claims 10 to 18, wherein the pure fungi biomass is mixed with a hydrocolloid solution, such as Xantham gum, optionally in an amount of from 0.1% to 5% of the total fungi biomass dry weight.

20. The method according to claim 19, where the hydrocolloid solution is added either during or after separation of the fungi biomass step in step c).

21. The method according to any one of claims 10 to 20, wherein in step a) a pH within the range of from 2.5 and 7.5 is maintained for the duration of the fermentation.

22. The method according to any one of claims 10 to 21, wherein the carbon source originates from a wheat flour-based substrate, optionally a wheat flour based substrate with minerals.

23. The method according to claim 22, wherein the amount of the wheat flour based substrate is within the range of from 5 g/L to 50 g/L dry weight based on the liquid substrate media.

24. The method according to any one of claims 10 to 23, wherein the pure fungi biomass subsequent to any one of steps c), d) is exposed to UV-B light, such as during a period of time of about 1 minute to 60 minutes.

25. Use of a pure fungi biomass comprising fermented fungi of a species belonging to the genus *Rhizopus* for preparing a food product in the form of a patty, nugget, burger, sausage, paste, chunk, fillet, extrudate, granules, cake, meat substitute, meat extender, jerky, fish-like product, seafood-like product, snack, beverage, dessert or baked goods.

FIG.1

FIG. 2

FIG. 3

FIG. 4

FIG. 5

FIG. 6

FIG. 7

FIG. 8

FIG. 9

FIG. 10

FIG. 11

FIG. 12

Europäisches
Patentamt

European
Patent Office

Office européen
des brevets

# EUROPEAN SEARCH REPORT

Application Number

**EP 21 18 3434**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 986 960 A1 (DSM NV [NL]) 22 March 2000 (2000-03-22) | 1-23,25 | INV. A23J3/22 |
| Y | * paragraphs [0014], [0018], [0020], [0021], [0026] – [0029], [0037], [0041], [0044] – [0055] * * paragraphs [0065] – [0081], [0087]; examples 2,4-19; tables 2-4 * | 24 | A23J1/00 A23L13/40 A23L31/00 C12N1/14 |
| Y | WO 2020/061502 A1 (THE BETTER MEAT COMPANY [US]) 26 March 2020 (2020-03-26) * paragraphs [0019] – [0021], [0028], [0034], [0036], [0049] – [0052], [0057], [0059] – [0062], [0064] – [0065], [0071] * * paragraphs [0103], [0104], [0108] – [0112], [0116], [0122] – [0129], [0143] – [0147], [0159]; claims 1,11,13,16,21,23-30, 41-42, 44-47, 49, 56-63, 70; figure 2; examples 1, 9 * | 1-25 | |
| Y | WO 2020/074782 A1 (TEKNOLOGIAN TUTKIMUSKESKUS VTT OY [FI]) 16 April 2020 (2020-04-16) * examples * | 1-25 | TECHNICAL FIELDS SEARCHED (IPC) A23J A23L C12R C12N |
| Y | WO 2014/004647 A1 (ENTIA BIOSCIENCES INC [US]) 3 January 2014 (2014-01-03) * page 23, lines 4-6 * * page 31, lines 22-25; example 3 * | 24 | |

The present search report has been drawn up for all claims

| Place of search | Date of completion of the search | Examiner |
|---|---|---|
| Munich | 15 December 2021 | Baminger, Ursula |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 18 3434

This annex lists the patent family members relating to the patent documents cited in the above-mentioned European search report.
The members are as contained in the European Patent Office EDP file on
The European Patent Office is in no way liable for these particulars which are merely given for the purpose of information.

15-12-2021

| Patent document cited in search report | | Publication date | Patent family member(s) | | | Publication date |
|---|---|---|---|---|---|---|
| EP 0986960 | A1 | 22-03-2000 | AT | 240660 | T | 15-06-2003 |
| | | | AU | 764133 | B2 | 14-08-2003 |
| | | | CA | 2336037 | A1 | 23-03-2000 |
| | | | DE | 69908132 | T2 | 04-03-2004 |
| | | | DK | 1094719 | T3 | 22-09-2003 |
| | | | EP | 0986960 | A1 | 22-03-2000 |
| | | | EP | 1094719 | A1 | 02-05-2001 |
| | | | ES | 2198151 | T3 | 16-01-2004 |
| | | | GB | 2357421 | A | 27-06-2001 |
| | | | JP | 2002524097 | A | 06-08-2002 |
| | | | NZ | 509053 | A | 20-12-2002 |
| | | | PT | 1094719 | E | 30-09-2003 |
| | | | US | 7045160 | B1 | 16-05-2006 |
| | | | WO | 0015045 | A1 | 23-03-2000 |
| | | | ZA | 200007697 | B | 13-07-2001 |
| WO 2020061502 | A1 | 26-03-2020 | CN | 113056202 | A | 29-06-2021 |
| | | | EP | 3852543 | A1 | 28-07-2021 |
| | | | US | 2020093155 | A1 | 26-03-2020 |
| | | | US | 2020093167 | A1 | 26-03-2020 |
| | | | WO | 2020061502 | A1 | 26-03-2020 |
| WO 2020074782 | A1 | 16-04-2020 | NONE | | | |
| WO 2014004647 | A1 | 03-01-2014 | CA | 2877861 | A1 | 03-01-2014 |
| | | | US | 2015157648 | A1 | 11-06-2015 |
| | | | WO | 2014004647 | A1 | 03-01-2014 |

EPO FORM P0459